Europäisches Patentamt

European Patent Office

Office européen des brevets

(11) Publication number: **0 352 139**
**A2**

## (12) EUROPEAN PATENT APPLICATION

(21) Application number: **89307464.1**

(22) Date of filing: **21.07.89**

(51) Int. Cl.⁵: **G 01 N 33/53**
**G 01 N 33/58, B 01 F 11/00**

(30) Priority: **21.07.88 GB 8817387**

(43) Date of publication of application:
**24.01.90 Bulletin 90/04**

(84) Designated Contracting States:
**BE CH DE FR GB IT LI NL SE**

(71) Applicant: **Lim, Pak Leong**
**Department of Microbiology University of Hong Kong**
**Hong Kong (HK)**

(72) Inventor: **Lim, Pak Leong**
**Department of Microbiology University of Hong Kong**
**Hong Kong (HK)**

(74) Representative: **Lynd, Michael Arthur et al**
**MARKS & CLERK 57 Lincoln's Inn Fields**
**London WC2A 3LS (GB)**

(54) **Detecting antigens or antibodies.**

㊼ A method for the detection of antigens or antibodies in a liquid sample, in which particles coated with antigen or antibody and having a diameter of from 50 to 1500 nm are used as the indicator reagent and are added to a set of micro-tubes containing the liquid sample in which antibody or antigen is to be detected, and in which method the tubes are incubated at ambient temperature with thorough mixing for a minimum period of 1 minute and sufficient to cause particles of the reagent to change if any of said antibody or antigen to be detected is present in the sample. The invention also offers a diagnostic kit for use in the detection of antigens or antibodies in a liquid sample, which kit comprises a set of micro-tubes for containing a said sample, and includes an indicator reagent comprising particles coated with antibodies or antigens responsive to the antigens or antibodies respectively to be detected in a said sample and having a diameter of from 50 to 1500 nm.

EP 0 352 139 A2

Description

The present invention relates to a method of, and to a kit for, detecting antigens or antibodies in samples, which samples may comprise body fluids or secretions or excretions, cells or tissue extracts, or culture media.

Antigen detection has long preoccupied immunologists working in diverse fields, ranging from food microbiology and experimental biology to, very importantly, the clinical sciences. Thus, tests are being increasingly employed to detect antigens in biological fluids for assisting with the diagnosis of infectious conditions, such as bacterial meningitis, typhoid fever and viral infections, and non-infectious conditions such as pregnancy and various hormonal disorders.

There has been a successive emergence of immunoassays for antigen detection over the last two decades. Radioimmunoassays (RIA) employing isotopic labels were popular in the 1960's, in which reactions were originally performed in solution. However, since the '70s, these have been largely superceded by the more convenient and safer solid-phase enzyme immunoassays (ELISA), which employ microplates, and enzyme labels instead of isotopic markers. A recently introduced variation of the RIA and ELISA systems is an immunoassay performed on a nitrocellulose membrane. These dot immunobinding assays, as well as the RIA and ELISA systems, are highly sensitive. However, they are all complicated as they involve multiple steps and, as conventionally performed, are also expensive and time-consuming. Simpler assays are available such as counter-immuneolectrophoresis, co-agglutination or slide latex agglutination, the methods most commonly used to detect antigens from cerebrospinal fluid specimens. Of these, slide latex agglutination is probably the most widely used today, due to its simplicity, low cost and speed. This is used to detect, among others, bacterial antigens in cerebrospinal fluid, viral particles in stool, and hormones in urine. In this method, a reagent comprising latex particles previously coated with an antibody or antigen is mixed with a small sample (usually less than 0.05 ml) of the test material on a slide or card, which is rotated or rocked gently for about five minutes. The result is then read visually, based on the presence or absence of particle-clumping. The main drawbacks of the method, however, are its relative insensitivity by comparison with the ELISA, and the fact that interpretation of the end-point can be subjective. Some improvement in these aspects can be achieved by performing the test in a large test-tube rather than on a slide. However, this tube method is seldom used for routine diagnosis except sometimes in cases of pregnancy or rheumatoid arthritis, because a large sample volume (1 ml) and a long incubation period (at least 1 hour) are required. In the instances where this has been used, the test is performed singly in individual large tubes, and the reaction mixture is not shaken during incubation.

Instead of antigens, antibodies are often used as the marker of infection or of other clinical conditions including the various auto-immune disorders. For this purpose, the total concentration of antibodies of a particular specificity in a serum sample is usually determined. However, diagnosis can be made more reliably if a particular class only of the antibodies present is measured. Usually, IgM antibodies are used, since these appear during the acute phase of the infectious disease and then usually disappear, unlike the IgG antibodies, when the infection is terminated. In rare situations, antibodies of other classes can also be diagnostically relevant. Thus, specific IgE antibodies were found in a study to be a better indicator of cytomegalovirus infection than IgM antibodies, while the presence of circulating IgA antibodies to the VCA antigen of Epstein-Barr virus is strongly correlated with the presence of nasopharyngeal carcinoma in a person.

The methods used for antibody detection are similar to those used for detecting antigens. The method most frequently used for determining class-specific antibodies is the ELISA, while RIA and immunofluorescence cytology are sometimes used. Conventional latex agglutination methods do not permit such a determination. However, antigen-coated latex particles have been used in an isolated instance to detect IgM antibodies against Rubella virus in a format based not on agglutination but on traditional solid-phase ELISA. The test was performed on microplates, and results were based on the settlement pattern of the latex particles in the microplate wells.

According to a first aspect of the present invention there is provided a method for the detection of antigens or antibodies in a liquid sample, in which particles coated with antigen or antibody and having a diameter of from 50 to 1500 nm are used as the indicator reagent and are added to a set of micro-tubes containing the liquid sample in which antibody or antigen is to be detected, and in which method the tubes are incubated at ambient temperature with thorough mixing for a minimum period of 1 minute and sufficient to cause particles of the reagent to change if any of said antibody or antigen to be detected is present in the sample.

According to a second aspect of the present invention there is provided a diagnostic kit for use in the detection of antigens or antibodies in a liquid sample, which kit comprises a set of micro-tubes for containing a said sample, and includes an indicator reagent comprising particles coated with antibodies or antigens responsive to the antigono or antibodies respectively to be detected in a said sample and having a diameter of from 50 to 1500 nm.

Preferably the particles used for coating with antibody or antigen (indicator particles) are monodisperse microspheres, which are composed, for example, of latex (e.g. styrene-butadiene copolymer, polystyrene, styrene-divinylbenzene copolymer or acrylic copolymer), or of a sol of gold or other materials, or mixtures of two or more thereof. In the case where a single species of particle is employed as the indicator reagent, the particles are preferably monosized. In the case of a latex, various different colours of particles can be used, but preferred colours are red, blue, green and yellow.

The particles are used as a suspension thereof either in a liquid carrier medium or directly in the liquid sample under assay. The concentration of the particle suspension used in the assay may vary from 0.001% (final, w/v) (for instrumental reading) to 0.2% (for visual examination). For most assays in which the results are read visually, a preferred concentration is from 0.01 to 0.08%, using particles preferably about 800nm in diameter.

The set of micro-tubes of the present invention is specially designed and is preferably arranged in a single row comprising two or more tubes (preferably from three to twelve, e.g. eight). The set of micro-tubes may be formed integrally, e.g. by casting from a single mould as a single unit or by machining tubes in a block of suitable material. Alternatively, the set of micro-tubes may be comprised of individual micro-tubes made separately but thereafter held more or less immovably in a holder therefor that allows the reaction contained within the micro-tubes to be observed. The set of two or more micro-tubes (of the same or different kinds) are thus physically linked or held together.

The material used for the set of micro-tubes is not of importance but should be at least translucent to allow the result of any reaction to be observed. Preferably the set of micro-tubes is cast from a plastics material.

Although the specific shape and dimensions of the set of micro-tubes and of the individual tubes is not critical, it is important that the tubes be relatively small. If the tubes are small then only a relatively small volume of reagent and sample is required.

Each micro-tube may hold from 0.05 to 0.5 ml of reaction mixture. Preferably, the mouth of each tube is just wide enough for easy delivery of the test material and reagents, and the tube tapers downwardly to a narrow width so that the reaction mixture can attain reasonable height in the tube for easy visual examination. Moreover, the tube preferably has sufficient depth to allow for weak concentrations of the reagent particles to be used and seen. The face of the set of micro-tubes is preferably flat for easy viewing of a reaction therethrough.

The preferred parameters for the shape and size of individual tubes, and of the set as a whole will be discussed hereinafter.

The concept of using sets of micro-tubes instead of large individual tubes is based on the following:-

a) It is easier to handle a number of small tubes physically joined together in a set than a single small tube.

b) Multiple (e.g. eight) tests can be performed simultaneously using a set of micro-tubes whereas it would be difficult to do more than (e.g.) three such tests with large, individual tubes.

c) The creation of a set of micro-tubes provides a new and simple mechanism for effective manual mixing the contents of the tubes which is otherwise hard to achieve with single tubes. (Note that in micro-tubes, the adhesive force between the contents of the tube and the tube can be considerable). The set of micro-tubes may be held at one end across the face between the thumb and the forefinger, and the set tapped repeatedly (e.g. 150 times per min.) against the palm of the other hand (or some other object), striking the face of the other end of the set against the object a few centimetres away from it. During this motion, the face of the set is preferably held horizontal. The force delivered to the contents of the tube will depend not only on the force and speed of motion applied, but also on the length and weight of the set, as well as the distance of the tube from the point where the set is held. The force is greatest in the tube farthest away. In practice, only the contents of tubes in the distal third will be effectively mixed this way, so that only one third of the set will be used at a time. Alternatively, the set of tubes may be placed in a particular holder (to be described hereinafter), which is at least thrice as long as the set, to attain the desired leverage for shaking the whole set when placed at the end of the holder.

d) Existing microtitre plates or strips consisting of series of wells joined together used for other immunoassay systems, are not suitable for use with particle immunoassays. These reaction systems are not designed to be shaken. For example, the desired mixing of the contents is not achieved by placing the microtitre plate on, for e.g., a flatbed mechanical rotator. There is no provision for sealing the wells of these vessels, essential in a mixing process. Moreover, reactions are viewed from the top through the mount of the wells, rather than through the side. The design of the wells of the prior art also differs from that of the set of micro-tubes of the present invention since in the former, results are based on the presence of absolute quantities of particular reactants, whereas particle immunoassays are based on the concentration (rather than absolute numbers) of the indicator particles.

It is essential to the present invention that the reaction mixture in the set of micro-tubes be thoroughly agitated for at least a minimum period of time. Such agitation causes any reaction to occur with far greater rapidity than is the case with the known method using individual large tubes. The speed and intensity of the reaction depend on the manner in ant the force with which the reaction mixture is shaken, as well upon the duration of the agitation. Best results are thus obtained using a mechanical mixer such as the one described hereinafter, using the preferred conditions described and preferably at least a five minute incubation period. Similar assay sensitivities, however, may be achieved by manual mixing for similar or even shorter periods, but with such manual mixing it is preferred that the micro-tubes be allowed to stand for a certain time (e.g. half-an-hour or overnight). (N.B. This "manual mixing + incubation" method is not equivalent to the known large tube method which uses merely static incubation. If static incubation only were to be effected upon samples in the set of micro-tubes of this invention, the results would be very poor and would be of low sensitivity.

The agitation of the set of micro-tubes of the present invention may be done manually or by means of a suitable mixer or agitation device. When manual agitation is employed, this may conveniently be effected using the method as hereinbefore described, in which the set of micro-tubes is held in one hand and then repeatedly struck against the other hand.

There is no mechanical mixer available which is designed to accommodate the sets of micro-tubes of the present invention, for mixing the tube contents to the extent required. When a mixer is used it should be of a special design. It may be capable of holding one or more sets of micro-tubes. The set(s) of micro-tubes may be mounted on one or both ends of an arm of the mixer which oscillates in a vertical plane about a horizontal axis. The range of the arm motion is at least 20° (preferably 47°) above and at least 20° (preferably 47°) below the horizontal. The speed of motion is at least 50 rpm, preferably 160 rpm.

Alternatively, there may be employed an electric mixer adapted to agitate the set of micro-tubes in a manner similar to that of the manual mixing device described hereinafter. Alternatively, a device which mixes the contents of the tubes in a different way, e.g. by repeated tube inversions may be employed.

The kit of the present invention may include the following items:-

1. A set of micro-tubes as hereinbefore defined, to be used as both the reaction and viewing vessel.

2. Reagents, including reaction indicator particles as hereinbefore defined and coated with the appropriate antigen or antibody.

3. Droppers, for delivering the test material to the tubes of the set of micro-tubes.

4. Accessories, including:

a) a holder for the set of tubes which can also be used for manually mixing the contents of the tubes.

b) an electric device which can hold one or more sets of tubes for mixing the contents of the tubes.

c) a viewer, consisting of an illuminator and a magnifying glass, for use in reading the results of the test by naked eye; and

d) an electronic device for reading the results of the test based on turbidometry, colorimetry, chemiluminescence or fluorimetry.

The kit is used and the method of the present invention performed, preferably in the following manner, preferably with all steps carried out at room temperature:-

The test sample (in e.g. 0.05 ml, suitably diluted if necessary, and with or without pretreatment) is placed together with a sample (e.g. 0.1 ml) of the indicator particles in one tube of the set of micro-tubes. The tube is then capped. Preferably control positive and negative samples are also included. The set of micro-tubes is then shaken either manually or mechanically to mix the contents thoroughly for a defined period or until the positive control shows the desired effect. The requirement of, and provision for, adequate mixing of the reactants is a cardinal feature of the present invention. Manual mixing may be effected by shaking the tubes continuously for from one to five minutes and, if necessary, repeating the process after an interval of a similar or longer period. The process can be repeated again for a few more times till a total time of e.g. from five to thirty minutes has elapsed.

Mechanical mixing may be continuously performed for from five to sixty minutes.

The results may be read immediately after the agitation process, or, for better resolution and sensitivity in particle agglutination systems, after a further period in which the tubes merely stand to allow the aggregated particles to settle. This subsequent incubation may be as short as a few minutes or, for greater sensitivity or for convenience, may be longer, e.g. 1 hour.

The results may be read by the naked eye or by an electronic device, viewing through a face of the set of micro-tubes. Visual examination is preferably based on a change of colour in the reaction mixture, achieved, for example, by using a combination of latex particles of two different colours so that reaction (settlement) of one type of particles leaves behind, in suspension, the particles of the other colour. Alternatively, in a monocolour system, results may be based on particle clumping (flocculation) or particle settlement (turbidometry). Instrumental reading of the results may be effected in various ways depending on the type of particles used. A colorimeter may be used to detect colour separation in multicoloured systems, whereas a spectrophotometer may be used to detect turbidometric changes in a monocolour system. It is envisaged that the particles may be made chemiluminescent or fluorescent, and that appropriate sensors based on such properties be used to detect the concentration of these particles.

The present invention is further illustrated in the following Examples:

EXAMPLE 1

Detection of antigens by direct method

In this method the test sample and the reagent particles are incubated together for a short time and the result then read on the presence or absence of agglutination (flocculation) in the reaction mixture. The assay can be performed in two ways, based on (a) the direct binding of the antigen in the sample by the reagent (antibody-coated) particles or (b) the competition between the antigen in the sample and the reagent particles (antigen-coated in this case) for the reagent antibody (in soluble form).

This method is only applicable to samples which do not interfere with particle agglutination, such as urine, cerebrospinal fluid, buffer extracts of cells (including microorganisms) and tissues, and culture media. Thus, the method can be used to detect bacterial antigens in culture broths (for identification purposes) or

4

cerebrospinal fluid (for the diagnosis of meningitis), or specifically <u>Trichinella spiralis</u> antigens from larval extracts derived from pork tissue, for the diagnosis of swine trichinellosis. Moreover, the competition method can be used to detect human chorionic gonadotropin (hCG) in urine for the diagnosis of both normal and ectopic pregnancy. Details of this latter application are set out below.

Method

The reagents were obtained from a commercially available pregnancy test kit used for performing slide latex agglutination tests (Gravindex β-hCG Slide Test for Pregnancy, Ortho Diagnostics Systems Inc, Raitan, NJ). These consisted of a suspension of latex particles which were covalently linked with hCG, and a solution or monoclonal antibodies specific for the β-subunit of hCG. Alternatively, these reagents could be prepared from base materials, e.g. using coloured latex particles (diameter, 800 nm) obtained from Rhone-Poulenic, Paris, hCG from Sigma Chemical Co., St. Louis, and monoclonal antibodies to β-hCG from Serotec, Oxford.

1. 0.1 ml of test material was placed in a tube.

2. 0.01 ml of a suitable dilution of the stock reagent antibody was added.

3. 0.01 ml of a suitable dilution of the indicator latex particles was added (final 0.01% w/v).

4. The tube was sealed and the set of tubes agitated by means of a mechanical mixer at room temperature for 30 min.

5. The results were then read visually.

Results

Using buffer (0.1 M glycine - 0.9% sodium chloride, pH 8.2) spiked with different amounts of hCG as test samples in the proposed method, the results obtained using different concentrations of reagent antibody for different periods of time are shown in the following Table 1. Thus, good sensitivity (31.3 int. units/L) was observed using 1:20 antibody dilution for 30 mins. This was 15-30 fold better than the sensitivity obtained with the conventional slide latex agglutination test done in parallel.

It was also observed in the study that the sensitivity of the proposed method was not affected by the reaction volume. Furthermore, thorough mixing of the reactants for a minimal time was found to be absolutely necessary, without which no reaction occurred at the concentrations of reactants used, even when the reaction mixture was incubated at 37°C for as long as 4 hours.

When the proposed method was applied to clinical specimens from patients suspected of being pregnant as well as from non-pregnant females, more cases could be detected than with the slide method, while the specificity remained the same. When the hCG levels in the positive cases were quantitated, the proposed method was 15-fold more sensitive than the slide method, although there was very good correlation between the two methods.

Table 1.

Effect of concentration of reagent antibody and length of incubation on the sensitivity of hCG detection by method of the present invention.

| hCG conc. (int. units/L) | Incubation period (min) | | | | | | | | | | | |
|---|---|---|---|---|---|---|---|---|---|---|---|---|
| | 15 | | | 30 | | | 45 | | | 60 | | |
| | antibody dilution | | | antibody dilution | | | antibody dilution | | | antibody dilution | | |
| | 1:10 | 1:20 | 1:40 | 1:10 | 1:20 | 1:40 | 1:10 | 1:20 | 1:40 | 1:10 | 1:20 | 1:40 |
| 125 | - | - | - | - | - | - | - | - | - | - | - | - |
| 62.5 | ± | - | - | ± | - | - | + | - | - | + | - | - |
| 31.3 | + | - | - | + | - | - | + | ± | - | + | ± | - |
| 16 | ++ | ± | - | ++ | ± | - | ++ | + | - | ++ | + | - |
| 8 | ++ | + | - | ++ | + | - | ++ | ++ | ± | ++ | ++ | ± |
| 4 | ++ | ± | - | ++ | + | - | ++ | ++ | + | ++ | ++ | + |
| 2 | ++ | + | - | ++ | + | - | ++ | ++ | + | ++ | ++ | + |
| 0 | ++ | + | ± | ++ | + | ± | ++ | ++ | + | ++ | ++ | + |

-, positive test (strong inhibition of agglutination).

±, weakly positive.

+, ++, negative test (agglutination).

(All based on visual examination)

EP 0 352 139 A2

EXAMPLE 2

Detection of antigens by capture method

This method is modified from the one described in Example 1 for use with clinical specimens such as serum which interfere with latex agglutination or with samples that are voluminous. It consists of two stages. In the first, antibody-coated beads are used to remove the antigen of interest from the test sample, and the beads are subsequently washed and re-suspended in buffer. In the second stage, indicator latex particles coated with the appropriate antibodies are added to the bead suspension to detect the relevant adsorbed antigen in a procedure similar to that described in Example 1. It is imperative that the beads settle to the bottom of the tube at the end of the experiment, while unreacted indicator particles remain suspended. This may be achieved by using beads of large sizes (e.g. 2000 nm), or with magnetic properties so that they can be sedimented by use of a magnet. The indicator particles, on the other hand, are small (less than 1200 nm) and will only sediment when bound to the beads through antigen-antibody interactions.

It is possible in this method to detect more than one type of antigens in the same test. To do this, beads coated separately with antibodies of the appropriate specificities are mixed together for use in antigen capture. The different types of coated beads used need not be distinguishable from one another. Secondly, indicator latex particles coated separately with the appropriate antibodies are used to develop the test. These must be distinguishable from one another, e.g., by colour. As an illustration, consider the detection of Rotavirus (RV) and Adenovirus (AV) for the differential diagnosis of infantile diarrhoea. If anti-RV and anti-AV indicator particles are used in the test and these are coloured, say, blue and yellow, respectively, the result of the test can be determined by colour development in the latex suspension. Thus, if only RV is present in the test sample, the latex suspension will appear more yellow at the end of the experiment compared to the original suspension, which is greenish, since only the blue anti-RV indicator particles have been removed to the bottom of the tube. Conversely, if only AV is present in the sample, the latex suspension appears blue, due to removal of the yellow anti-AV particles. On the other hand, if only one type of indicator latex particles is ued, the results can be determined by turbidometric measurement as in Example 1. This latter application is described in the study below for the detection of Salmonella typhi lipopolysaccharide using an 0-9 monoclonal antibody produced in our laboratory.

Method

Magnetic beads (Dynabeads M-450; diameter, 4500 nm) were obtained from Dynal, Skoyen, Oslo, and latex particles (diameter, 800 nm) obtained from Sigma Chemical Co., St. Louis. Both particles were coated with a monoclonal antibody to Salmonella 0-9 antigen. The antigen used, S. typhi lipopolysaccharide (LPS), was bought from Difco Co.

1. 0.05 ml of the antibody-coated Dynabeads (of a suitable concentration) was placed in a tube in the set.

2. 0.1 - 5.0 ml of test sample (LPS-spiked buffer) were added to the tube.

3. The tube was sealed and the set of micro-tubes agitated by a mechanical mixer at room temperature for 45 min.

4. The beads were washed three times with buffer and collected at the bottom of the tube by means of a magnet.

5. To the beads was added 0.1 ml of the indicator latex reagent (final concentration, 0.01% w/v).

6. The mixture was incubated as in step 3.

7. The Dynabeads were collected at the bottom of the tube and the result of the test then read.

Results

The following Table 2 shows the results of antigen detection using the proposed capture method, compared to the direct one-step procedure described in Example 1. The direct method was more sensitive than the capture method when the same volume (0.1 ml) of test sample was used in both. However, with bigger volumes of test material, the sensitivity of the latter method increased significantly while that of the former remained the same. The sensitivity of the capture method was influenced by the period of agitation/incubation at both stages of the test. Not illustrated is the greater inhibitory effect of serum on the direct method than on the capture method.

Table 2.

Antigen detection by capture method[a]

| Vol test sample (ml) | Antigen conc. (ng/ml) | | | | |
|---|---|---|---|---|---|
| | 50 | 25 | 12.5 | 6.25 | 0 |
| 0.1(direct method)[b] | + | ± | - | - | - |
| 0.1 | ±(84) | -(100) | -(100) | -(96) | - |
| 0.5 | -(89) | ±(85) | -(100) | -(98) | - |
| 2.0 | +(58) | +(63) | +(68) | -(99) | - |
| 5.0 | +(46) | +(60) | +(66) | +(68) | - |

[a] Visual examination: +, positive test; -, negative test; ±, weakly positive. Figures in parenthesis denote turbidity of reaction mixture as measured in a spectrophotometer (400 nm) expressed as a % of turbidity in control tubes.
[b] A 1-step method using the same indicator latex particles but without the capture-beads.

EXAMPLE 3

Detection of IgM antibodies to infectious agents by capture method

The method here is similar to that described in Example 2. Firstly, antibody (anti-μ)-coated beads are used to remove total IgM antibodies from the test (serum) sample. Secondly, the presence of antibodies of a particular specificity in the adsorbed antibodies is determined using indicator latex particles coated with the relevant antigen. It is possible to detect antibodies of more than one specificity in the same test. This involves the use of indicator particles of different colours, each coated with a different antigen. For example, to determine whether the cause of viral hepatitis in a patient is due to Hepatitis A virus (HAV) or Hepatitis B virus (HBV), HAV and HBV indicator particles coloured, say, blue and yellow, respectively, are added to the IgM-bound beads. If IgM anti-HAV is present, then the resulting latex suspension at the end of the experiment will appear yellow compared to the greenish colour of the original or control suspensions, since the blue HAV indicator particles will have become bound to the sedimented beads. Conversely, if IgM anti-HBV is present, the latex suspensions become bluish.

As an illustration, the method of the present invention was used in the study described below for the detection of IgM antibodies to Trichinella spiralis in mouse sera.

Method

Dynabeads (Dynal, Oslo) coated with purified goat anti-μ (mouse) antibodies (Sigma), and latex particles (800 nm; Sigma) covalently conjugated with T. spiralis antigen were used in place of the corresponding reagents in method of Example 2. Otherwise, the procedures described were the same.

Results

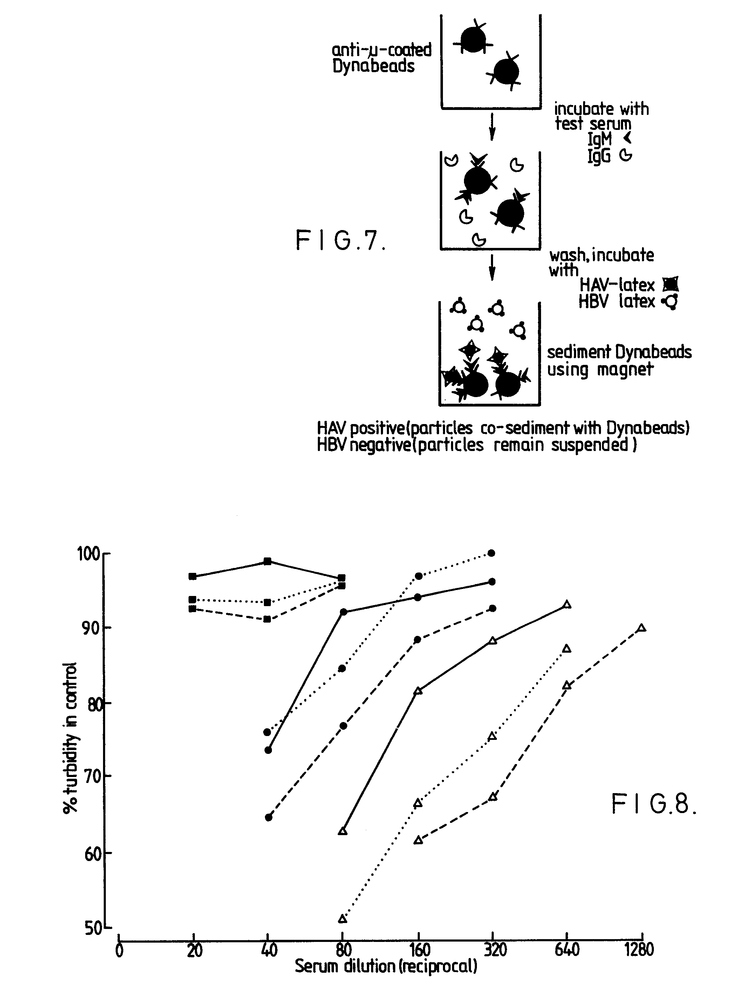

The Graph of Figure 8 shows the results of specific IgM detection by the proposed method. The results were read using a spectrophotometer set at a wavelength of 400 nm, and are expressed as a % of the turbidity observed in control, unreacted latex suspensions. A decrease of 20% or more in turbidity is discernable by eye. In the figure shown, serial dilutions of nine serum samples were examined in the test. If 80% turbidity is used as the cut-off level, then six of the samples were positive in the test, with titres of specific IgM antibodies ranging from 1:50 to 1:600. There was good correlation with results obtained of these sample by an enzyme-linked immunosorbent assay.

The present invention is further illustrated in the accompanying drawings wherein:
Figure 1 shows embodiments of a set of micro-tubes of the present invention,
Figure 2 shows a further embodiment of a set of micro-tubes according to the present invention,
Figure 3 shows a sketch of a holder for manually agitating a set of micro-tubes according to the present invention,
Figure 4 shows a vertical section through a mixer which can be used in the method of the present invention,

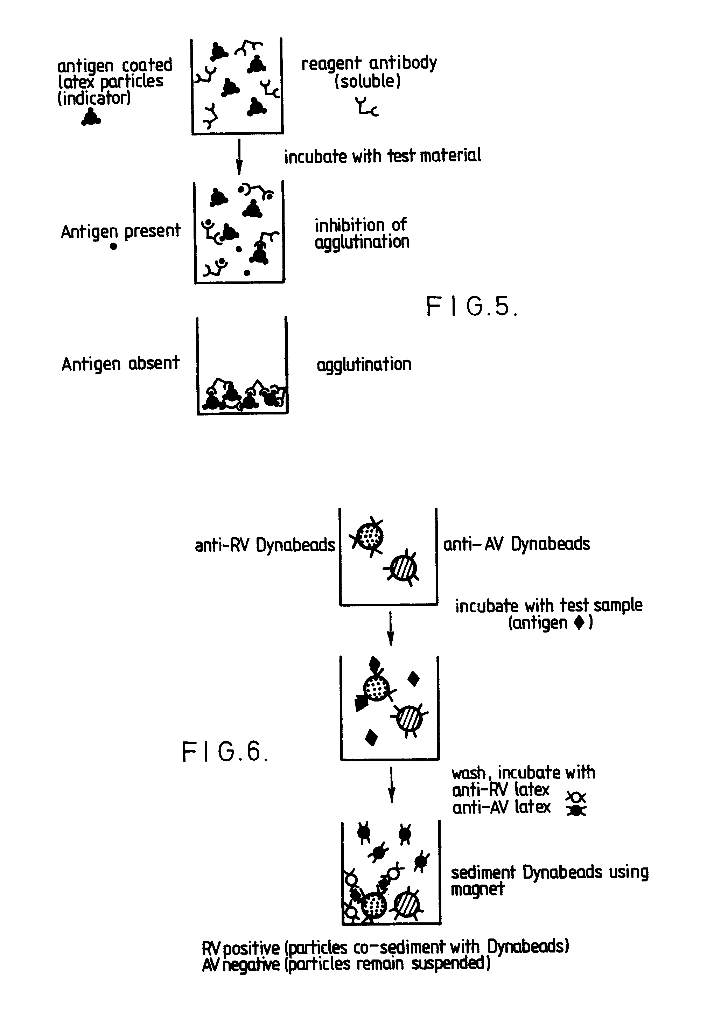

Figure 5 illustrates the method of Example 1,
Figure 6 illustrates the method of Example 2,
Figure 7 illustrates the method of Example 3 and
Figure 8 is a graph showing the results of Example 3.

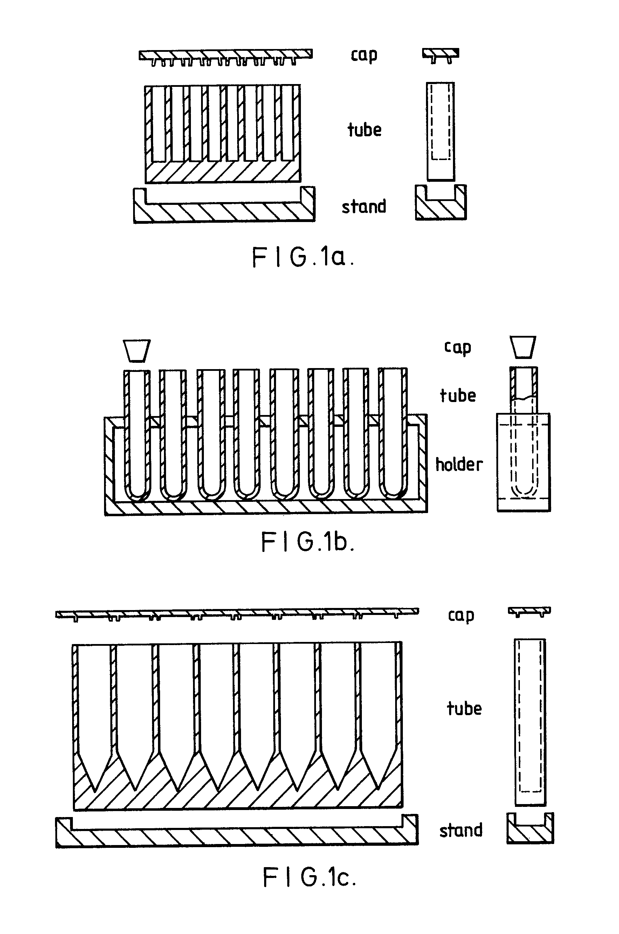

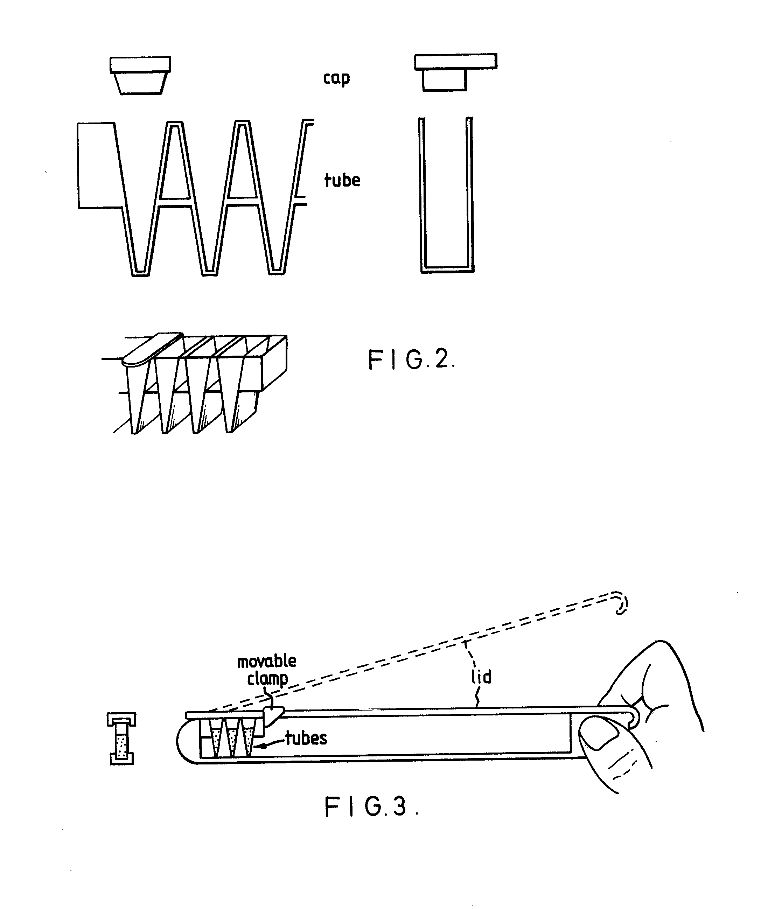

Referring firstly to the sets of micro-tubes shown in Figs 1 and 2, it is believed that the Figures are essentially self-explanatory. In Figs 1(a), 1(c) and Fig. 2, the set of micro-tubes is formed as an integral unit, e.g. by injection moulding.

In Fig. 1 (b) individual micro-tubes are held or clamped in a holder therefor. In the embodiment of Fig. 2, the micro-tubes are spaced at 1 cm centres and have a height of approximately 2 cm. The width of the set of micro-tubes of Fig. 2 is 1 cm and the individual micro-tubes of the set each tapers from a wide mouth to a narrow base.

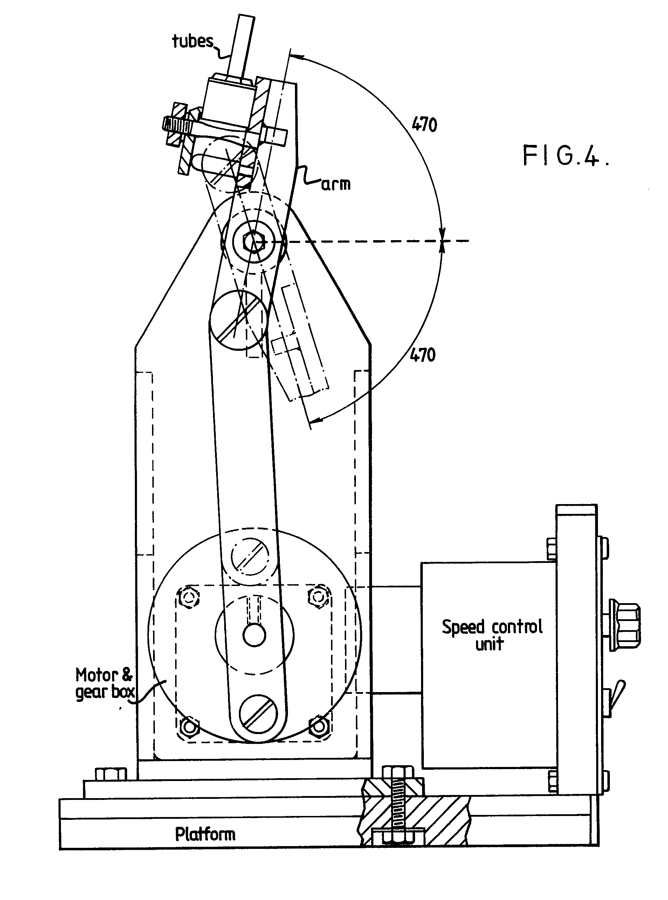

Referring now to Fig. 3 of the drawings, there is shown a simple holder and mixer for a set of micro-tubes according to the present invention. The tube holder-mixer is designed to hold a set of tube of wide-ranging length (or even a single tube) so that the tubes can be shaken at one end of the holder-mixer while the other end is held by a hand. The principle and procedure used are based on those used in the set of tubes.

Instead of using the holder of Fig. 3 it is possible very easily to improvise a suitable holder for manual mixing. For example, it might be possible merely to use a ruler, in which case the set of tubes could be merely clipped on to one of its ends.

The kit of the present invention differs from existing diagnostic kits which also utilise particles as reaction indicators in at least the following ways:-

1. The reaction vessel used is different. A set of specially designed micro-tubes is used, as opposed to individual tubes, which allows multiple tests to be performed simultaneously and which also enables, by virtue of its structure, the contents of the tubes to be mixed effectively when manual agitation is used. In contrast, slides or cards (slide latex agglutination test) or single (large) tubes (tube latex agglutination test) have been employed in known kits.

2. The incubation conditions used are different. There is an obligatory step in the method of the present invention in which the contents of the tubes have to be thoroughly mixed for at least one minute, using a particular method of mixing (manual) or using a specially designed device (mechanical). Existing tube latex agglutination tests do not use such a step, which has been considered unnecessary since the reagent particles used in the assay are believed small enough in size to be able to move by Brownian motion alone or under mere thermal agitation (i.e. by incubation at 37°C).

3. In some embodiments of the method of the present invention the test sample is pretreated, for example, with a reagent particle (Dynabeads), before incubation with the indicator particles. This two-particle system has not been used before.

4. Results based on colour separation when combinations of indicator particles of different colours are used have not been used in tube tests before. A similar method of analysis, however, has been used in a slide latex agglutination test, but the pattern of colour separation observed here is different from that of the proposed tube method. A scheme based on a colour change of the reaction mixture has also been used for large individual tubes, employing a single type of non-latex (gold sol) particles; the mechanism of colour change in this system, however, is different from that of the two-particle system.

## Claims

1. A method for the detection of antigens or antibodies in a liquid sample, in which particles coated with antigen or antibody and having a diameter of from 50 to 1500 nm are used as the indicator reagent and are added to a set of micro-tubes containing the liquid sample in which antibody or antigen is to be detected, and in which method the tubes are incubated at ambient temperature with thorough mixing for a minimum period of 1 minute and sufficient to cause particles of the reagent to change if any of said antibody or antigen to be detected is present in the sample.

2. A method according to Claim 1, wherein the particles comprise latex particles.

3. A method according to Claim 2, wherein a mixture of latex particles of two or more different colours are employed.

4. A method according to Claim 1, wherein the sample is pre-treated with a second species of particles, different from and separable from the indicator particles.

5. A method according to Claim 4, wherein the second species of particles is differentiable from the indicator by being magnetic or by being of larger size.

6. A method according to Claim 1, wherein the agitation of the set of micro-tubes is effected manually.

7. A method according to Claim 6, wherein manual mixing is employed.

8. A method according to Claim 1 wherein the agitation of the set of micro-tubes is effected by machine.

9. A method according to Claim 8, wherein the agitation of the set of microtubes is effected by moving the set of tubes up and down on the end of an arm oscillating about a horizontal axis.

10. A method according to Claim 9, wherein the oscillating arm moves over an angular range of from at least +20° to at least -20° from the horizontal.

11. A method according to Claim 9, wherein the arm oscillates at at least 50 strokes per minute.

12. A method according to Claim 1, wherein the change, if any, is detected visually.

13. A method according to Claim 1, wherein the change, if any, is detected by one of spectrometry, colorimetry, chemiluminescence and fluorimetry.

14. A method according to Claim 1, the change, if any, is detected by colour separation.

15. A diagnostic kit for use in the detection of antigens or antibodies in a liquid sample, which kit comprises a set of micro-tubes for containing a said sample, and includes an indicator reagent comprising particles coated with antibodies or antigens responsive to the antigens or antibodies respectively to be detected in a said sample and having a diameter of from 50 to 1500 nm.

16. A kit according to Claim 15, wherein the set of microtubes is substantially as shown in Fig. 1(a), Fig. 1(b) or Fig. 1(c) of the accompanying drawings.

17. A kit according to Claim 15, wherein the set of micro-tubes is substantially as shown in Fig. 2 of the accompanying drawings.

18. A kit according to Claim 15, which includes a device for manually mixing the sample in the set of micro-tubes.

19. A kit according to Claim 18, wherein the device is substantially as shown in Fig. 3 of the accompanying drawings.

20. A kit according to Claim 14, which includes a mechanical mixer for mixing a sample in the set of micro-tubes.

21. A machine for agitating a set of micro-tubes in a method of detecting antigens or antibodies, which machine includes an arm adapted to oscillate about a horizontal axis, means for causing the arm so to oscillate, and further means, located at a distal end of the arm, for holding a said set of micro-tubes.

22. A machine for agitating a set of microtubes in a method of detecting antigens or antibodies, which machine is substantially as illustrated in Fig. 4 of the accompanying drawings.

cap

tube

stand

F I G .1a.

cap

tube

holder

F I G .1b.

cap

tube

stand

F I G .1c.

FIG.2.

FIG.3.

tubes

arm

470

470

F I G.4.

Speed control unit

Motor & gear box

Platform

antigen coated
latex particles
(indicator)

reagent antibody
(soluble)

incubate with test material

Antigen present

inhibition of
agglutination

F I G.5.

Antigen absent

agglutination

anti-RV Dynabeads

anti-AV Dynabeads

incubate with test sample
(antigen ♦)

F I G.6.

wash, incubate with
anti-RV latex
anti-AV latex

sediment Dynabeads using
magnet

RV positive (particles co-sediment with Dynabeads)
AV negative (particles remain suspended)

anti-μ-coated Dynabeads

incubate with test serum
IgM ⊰
IgG ↺

F I G.7.

wash, incubate with
HAV-latex ▨
HBV latex ⊘

sediment Dynabeads using magnet

HAV positive (particles co-sediment with Dynabeads)
HBV negative (particles remain suspended)

F I G.8.